# EUROPEAN PATENT SPECIFICATION

(11) **EP 2 671 874 B1**
(45) Date of publication and mention of the grant of the patent: **13.01.2016**
(21) Application number: 12171097.4
(22) Date of filing: 06.06.2012
(51) Int. Cl.: C07D 231/14

(54) **Preparation of esters of 1-substituted-3-fluoroalkyl-pyrazole-4-carboxylic acids**
Herstellung von 1-substitutierten 3-Fluoralkyl-pyrazol-4-carboxylsäure-Estern
Fabrication d'esters de 3-fluoroalkyl-pyrazole-4-carboxylique substitués en position 1

(43) Date of publication of application: 11.12.2013
(73) Proprietor: SOLVAY SA, 1120 Bruxelles (BE)
(72) Inventor: Jaunzems, Janis, 30559 Hannover (DE)
(74) Representative: Mross, Stefan P.M.

(56) References cited:
- WO-A1-2012/010692
- VALENTINA DICHIARANTE ET AL: "Eco-friendly hydrodehalogenation of electron-rich aryl chlorides and fluorides by photochemical reaction", GREEN CHEMISTRY, vol. 11, no. 7, 1 January 2009 (2009-01-01), page 942, XP55037160, ISSN: 1463-9262, DOI: 10.1039/b904897a

## Description

The invention concerns a process for the manufacture of esters of 1-substituted-3-fluoroalkyl-pyrazole-4-carboxylic acid, in particular esters of 3-difluoromethyl-1-methyl- 1H-pyrazole-4-carboxylic acid, which are useful e.g. as intermediates for pharmaceuticals and agrochemicals.

US patent 5,498,624 describes the preparation of 3-difluoromethyl-1-methyl- 1H-pyrazole-4-carboxylic acid derivatives which are intermediates for the manufacture of pyrazole carboxanilide fungicides.

WO 2008/053043 discloses a process for the synthesis of difluoromethyl - substituted-pyrazole-4-carboxylic acid esters. The synthesis is carried out by reacting 4,4,4-trihalogen-substituted acetoacetic ester derivatives with chlorosilanes in the presence of magnesium or other metals of the 1 st, 2nd, 3rd, 4th or 12th group of the Periodic Table of the Elements and subsequent reaction of the reaction product with a hydrazine or hydrazine WO 2012/010692 discloses the preparation of 3-difluoromethyl-pyrazole-4-carboxylates from the corresponding 3-chlorodifluoromethyl-derivatives in the presence of a reducing agent.

It is an object of the present invention to provide a process for the synthesis of esters of 1-substituted-3-fluoroalkyl-pyrazole-4-carboxylic acid which allows for high efficiency, and, in particular, high selectivity and for an environmental beneficial process. The process according to the invention also allows the utilization of starting materials (e.g.chlorodifluoroacetyl chloride (CDFAC) which are available in industrial scale.

The invention consequently relates to a process for the manufacture of an ester of a 1-substituted-3-fluoroalkyl-pyrazole-4-carboxylic acid of formula (I) 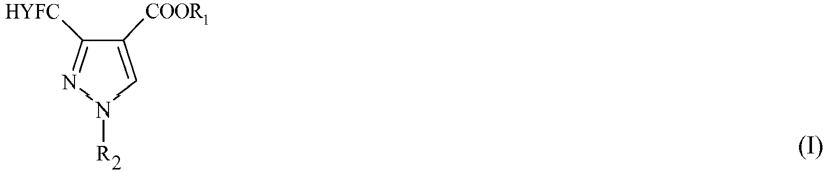 wherein
- Y is H, F or an alkyl group having from 1 to 12 carbon atoms which is optionally substituted by at least one halogen atom, an aralkyl group or an aryl group ;
- R₁ is H or an organic residue,
- R₂ is H or an organic residue,
which comprises submitting a compound of formula (II) 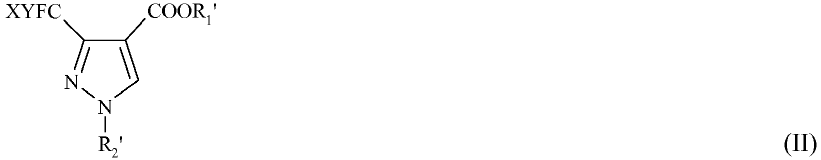 wherein Y is as defined above
- X is Cl, Br or I,
- R₁' is H or an organic residue,
- R₂' is H or an organic residue,
to a reduction reaction supported by UV light. The reduction reaction is a dehydrohalogenation reaction.

The term "ester" includes, for the sake of simplicity, the free acid ; this is the case when R₁' is H. The esters are preferred ; i.e., R₁' preferably is an organic residue.

It should be noted that R₁' can be identical to or different from R₁. Also R₂' can be identical to or different from R₂. R₁' and/or R₂' are different from R₁ and R₂, if R₁' and/or R₂' undergo a reduction reaction in the process according to the invention. If reduction of the R₁' and/or R₂' occurs, the resulting R₁ and R₂ can be defined as the reduced groups of R₁' and/or R₂' respectively.

The term "organic residue" is intended to denote in particular linear or branched alkyl or alkylene groups which may contain hetero atoms, such as in particular boron, silicon, nitrogen, oxygen or sulphur atoms and halogen atoms, cycloalkyl groups or cycloalkylene groups, heterocycles and aromatic systems. The organic residue may contain double or triple bonds and functional groups.

The organic residue comprises at least 1 carbon atom. It often comprises at least 2 carbon atoms. It preferably comprises at least 3 carbon atoms. More particularly preferably, it comprises at least 5 carbon atoms.

The organic residue generally comprises at most 100 carbon atoms. It often comprises at most 50 carbon atoms. It preferably comprises at most 40 carbon atoms. More particularly preferably, it comprises at most 30 carbon atoms.

R₁ is typically selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof.

R₂ is usually selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof.

R₁' is generally selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof.

R₂' is most often selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof.

The term "alkyl group" as given in the definition of organic residue is intended to denote in particular a linear or branched alkyl substituent comprising from 1 to 20 carbon atoms, preferably 1, 2, 3, 4, 5, 6, 7, 8, 9 or 10 carbon atoms. Specific examples of such substituents are methyl, ethyl, n-propyl, isopropyl, n-butyl, isobutyl, tert-butyl, n-pentyl, isopentyl, n-hexyl, 2-hexyl, n-heptyl, n-octyl and benzyl.

The term "cycloalkyl group" is intended to denote in particular a substituent comprising at least one saturated carbocycle containing 3 to 10 carbon atoms, preferably 5, 6 or 7 carbon atoms. Specific examples of such substituents are cyclopropyl, cyclobutyl, cyclopentyl, cyclohexyl and cycloheptyl.

The term "alkylene group" or "cycloalkylene group" is intended to denote in particular the divalent radicals derived from the alkyl or cycloalkyl groups as defined above.

When the organic residue contains one or optionally more double bonds, it is often chosen from an alkenyl group comprising from 2 to 20 carbon atoms, preferably 2, 3, 4, 5, 6, 7, 8, 9 or 10 carbon atoms or a cycloalkenyl group comprising from 3 to 20 carbon atoms, preferably 3, 4, 5, 6, 7, 8, 9 or 10 carbon atoms. Specific examples of such groups are vinyl, 1-allyl, 2-allyl, n-but-2-enyl, isobutenyl, 1,3-butadienyl, cyclopentenyl, cyclohexenyl and styryl.

When the organic residue contains one or optionally more triple bonds, it is often chosen from an alkinyl group comprising from 2 to 20 carbon atoms, preferably 2, 3, 4, 5, 6, 7, 8, 9 or 10 carbon atoms. Specific examples of such groups are ethinyl, 1-propinyl, 2-propinyl, n-but-2-inyl and 2-phenylethinyl.

When the organic residue contains one or optionally more aromatic systems, it is often an aryl group comprising from 6 to 24 carbon atoms, preferably from 6 to 12 carbon atoms. Specific examples of such groups are phenyl, 1-tolyl, 2-tolyl, 3-tolyl, xylyl, 1-naphthyl and 2-naphthyl.

The term "heterocycle" is intended to denote in particular a cyclic system comprising at least one saturated or unsaturated ring made up of 3, 4, 5, 6, 7 or 8 atoms, at least one of which is a hetero atom. The hetero atom is often chosen from B, N, O, Si, P and S. It is more often chosen from N, O and S.

Specific examples of such heterocycles are aziridine, azetidine, pyrrolidine, piperidine, morpholine, 1,2,3,4-tetrahydroquinoline, 1,2,3,4-tetrahydroisoquinoline, perhydroquinoline, perhydroisoquinoline, isoxazolidine, pyrazoline, imidazoline, thiazoline, tetrahydrofuran, tetrahydrothiophene, pyran, tetrahydropyran and dioxane.

The organic residues as defined above may be unsubstituted or substituted with functional groups. The term "functional group" is intended to denote in particular a substituent comprising or consisting of a hetero atom. The hetero atom is often chosen from B, N, O, Al, Si, P, S, Sn, As and Se and the halogens. It is more often chosen from N, O, S and P, in particular N, O and S.

The functional group generally comprises 1, 2, 3, 4, 5 or 6 atoms.

By way of functional groups, mention may, for example, be made of halogens, a hydroxyl group, an alkoxy group, a mercapto group, an amino group, a nitro group, a carbonyl group, an acyl group, an optionally esterified carboxyl group, a carboxamide group, a urea group, a urethane group and the thiol derivatives of the abovementioned groups containing a carbonyl group, phosphine, phosphonate or phosphate groups, a sulphoxide group, a sulphone group and a sulphonate group.

In a preferred embodiment of the process according to the invention, R₁ is H, C₁-C₈-alkyl, C₃-C₈-cycloalkyl, C₁-C₄-alkoxy-C₁-C₄-alkyl, C₃-C₈-cycloalkoxy-C₁-C₄-alkyl, C₂-C₈-alkenyl or is benzyl which is optionally substituted by 1,2 or 3 substituents R^{Y1} independently of one another selected from the group consisting of C₁-C₄-alkyl, C₁-C₄-alkoxy and nitro ; and

R₂ is hydrogen, C₁-C₄-alkyl, benzyl or phenyl, where the two last-mentioned substituents may be unsubstituted or optionally substituted by 1,2 or 3 substituents R^{Y2} independently of one another selected from the group consisting of halogen, nitrile, nitro, C₁-C₄-alkyl, C₁-C₄-haloalkyl, C₁-C₄-alkoxy and C₁-C₄-haloalkoxy ; and
X is Cl.

The terms, used in the definition of the variables, for organic groups, such as, for example, the term "halogen", are collective terms representing the individual members of these groups of organic moieties.

The prefix Cₓ-C_{y} denotes the number of possible carbon atoms in the case in question. C₁-C₄-Alkyl includes, for example, methyl, ethyl, propyl, 1-methylethyl, butyl, 1-methylpropyl, 2-methylpropyl or 1,1-dimethylethyl.

The term "halogen" denotes in each case fluorine, bromine, chlorine or iodine, especially fluorine, chlorine or bromine.

The term "C₁-C₄-alkoxy-C₁-C₄-alkyl", as used herein, describes C₁-C₄-alkyl radicals where one carbon atom is attached to a C₁-C₄-alkoxy radical. Examples of these are CH₂-OCH₃, CH₂-OC₂H₅, n-propoxymethyl, CH₂-OCH(CH₃)₂, n-butoxymethyl, (1-methylpropoxy)methyl, (2-methylpropoxy)methyl, CH₂-OC(CH₃)₃, 2-(methoxy)ethyl, 2-(ethoxy)ethyl, 2-(n-propoxy)ethyl, 2-(1-methylethoxy)ethyl, 2-(n-butoxy)ethyl, 2-(1-methylpropoxy)ethyl, 2-(2-methylpropoxy)ethyl, 2-(1, 1 -dimethylethoxy)ethyl, 2-(methoxy)propyl, 2-(ethoxy)propyl, 2-(n-propoxy)propyl, 2-(1-methylethoxy)propyl, 2-(n-butoxy)propyl, 2-(1-methylpropoxy)propyl, 2-(2-methylpropoxy)propyl, 2-(1,1-dimethylethoxy)propyl, 3-(methoxy)propyl, 3-(ethoxy)propyl, 3-(n-propoxy)propyl, 3-(1-methylethoxy)propyl, 3-(n-butoxy)propyl, 3-(1-methylpropoxy)propyl, 3-(2-methylpropoxy)propyl, 3-(1,1-dimethylethoxy)propyl, 2-(methoxy) butyl, 2-(ethoxy)butyl, 2-(n-propoxy)butyl, 2-(1-methylethoxy)butyl, 2-(n-butoxy)butyl, 2-(1-methylpropoxy)butyl, 2-(2-methylpropoxy)butyl, 2-(1,1-dimethylethoxy)butyl, 3-(methoxy)butyl, 3-(ethoxy)butyl, 3-(n-propoxy)butyl, 3-(1-methylethoxy)butyl, 3-(n-butoxy)butyl, 3-(1-methylpropoxy)butyl, 3-(2-methylpropoxy)butyl, 3-(1,1-dimethylethoxy)butyl, 4-(methoxy)butyl, 4-(ethoxy)butyl, 4-(n-propoxy)butyl, 4-(1-methylethoxy)butyl, 4-(n-butoxy)butyl, 4-(1-methylpropoxy)butyl, 4-(2-methylpropoxy) butyl, 4-(1,1-dimethylethoxy)butyl, etc.

The term "C₂-C₈-alkenyl", as used herein, describes straight-chain and branched unsaturated hydrocarbon radicals having 2 to 8 carbon atoms and at least one carbon-carbon double bond, such as, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-l-methyl-2-propenyl, 1-ethyl-2-methyl-1-propenyl and 1-ethyl-2-methyl-2-propenyl, 1-heptenyl, 2-heptenyl, 1-octenyl or 2-octenyl.

In a preferred embodiment of the process according to the invention,
R₁ is H, C₁-C₄-alkyl or benzyl, in particular methyl, ethyl, n-propyl or isopropyl ;
R₁ is especially ethyl; and
R₂ is H or C₁-C₄-alkyl. R₂ is especially methyl ; X is C1 and Y is F.

The reduction process according to the invention comprises reacting the compound of formula (II) with a hydrogen donating compound. The term "hydrogen donating compound" denotes compounds which provide H atoms which react with the CFXY group wherein X is Cl, Br or I under substitution of the halogen atom X by a hydrogen atom H thus forming a CHFY group, and consequently, the desired compound of formula (I).

H₂O is a suitable hydrogen source.

The hydrogen donating compound preferably is selected among organic compounds which are susceptible to oxidation by a UV light induced radical reaction. For example, the hydrogen donating compound may be selected from the group consisting of alcohols, ethers, ketones, hydrocarbons and esters and other compounds which bear an aliphatic C-H moiety. Preferably, the hydrogen containing compound has a melting point equal to or lower than 50°C. Especially preferably, it is liquid in the range from -20°C to +50°C.

Preferably, the hydrogen donating compound is selected from linear or branched monobasic alcohols with 1 to 5 C atoms and linear or branched dibasic alcohols with 2 to 5 C atoms. Preferably, monobasic alcohols with 1 to 3 C atoms are applied as hydrogen donating compound. Isopropanol is especially preferred.

If desired, additional solvents may be present, for instance polar aprotic solvents like tetrahydrofurane (THF), ethyl acetate, acetone, dimethylformamide (DMF), acetonitrile or dimethylsulfoxide (DMSO), especially THF. A single polar solvent can be used or a mixture of two or more polar solvents.

In the process according to the invention, the hydrogenation reaction is carried out in the liquid phase. The compound of formula (II) is advantageously dissolved in a solvent. Examples of polar solvents comprising at least one OH group may be selected from a group consisting of aliphatic alcohols preferably comprising from 1 to 3 carbon atoms. Aliphatic alcohols preferably comprising from 1 to 3 carbon atoms such as methanol, ethanol, isopropanol (IPA) and the like are preferred as solvents. Especially preferably, the hydrogen donating compound is also solvent for the starting compound of formula (II). Thus, methanol, ethanol, isopropanol (IPA) are especially preferred because they may function as solvent and hydrogen donating compound.

Optionally, the reduction process comprises substituting halogen, in particular chlorine atom by a hydrogen atom and simultaneously hydrogenation of at least one of the unsaturated bonds in the substituents R₁' and/or R₂'.

The reaction between the compound of formula (II) and the hydrogen donating compound is performed in the presence of UV light. The UV light may be intermittently radiated into the reaction mixture, or continuously. Any radiation with a wavelength equal to or lower than 254 nm can be applied to provide the energy needed to hydrogenate the CFXY group. UV light sources emitting light having one or more emissions with a wavelength equal to or greater than 210 and equal to or lower than 254 nm are especially suitable.

Especially preferably, the UV light source is selected from middle or low pressure mercury lamps. If desired, LEDs and OLEDs emitting radiation in the UV spectrum can be applied as UV light source.

The hydrogen donating compound is typically used in an amount of equal to or greater than 1 mol equivalent compared to the content of compound of formula (II). Preferably, an excess of the hydrogen donating compound is applied ; it may serve as a solvent. The amount of the hydrogen donating compound may be lower than stoichiometrically needed but then the yield may be lower.

In the hydrogenation reaction, the temperature of the reaction is generally higher that the melting point of the hydrogen donating compound and the solvent. Often, the reaction temperature is equal to or greater than -20°C, preferably, it is equal to or greater than -10°C. The temperature of the reaction more preferably is equal to or greater than 0°C. Preferably, the temperature is equal to or lower than 100°C, more preferably equal to or lower than 50°C. Performing the reaction at ambient temperature is very particularly preferred.

The reaction is preferably performed at ambient pressure. If desired, it could also be performed under reduced pressure or at a pressure above ambient pressure.

In the hydrogenation reaction, the concentration of the compound of formula II in the reaction medium is generally at least 5 % by weight with respect to the total weight of the reaction medium. This concentration is often at least 10 % by weight. Preferably, the concentration is at least 20 % by weight. The concentration of the compound of formula II in the reaction medium is generally at most 50 % by weight with respect to the total weight of the reaction medium.

In the process according to the invention, the reduction reaction may be carried out in the presence of at least one additive, especially of an organic base, an inorganic base, more particularly an inorganic base. Organic bases are for instance ammonia or amines and their salts with organic acids or the salts of alkali metals with organic acids. Suitable are, for example, primary, secondary and tertiary amines, e.g. triethylamine, ammonium acetate (NH₄OAc), and sodium acetate (NaOAc). Inorganic bases can for instance be selected from K₂C0₃, Cs₂CO₃, Na₂C0₃, NaHCO₃, K₃PO₄, NaOH, and KOH.

If an additive or base is used, then said additive or base is typically added in an amount of from 0.05 to 5 molar equivalents of compound of formula (II), often from 0.1 to 3, more often from 0.5 to 2, for instance around 1.

Preferably, the reduction is carried out without addition of any additives and/or bases.

The process according to the invention allows avoiding with particular efficiency the over-reduction of other halogen substituents, especially of fluorine atoms. The process according to the invention can be used for the manufacture of industrial amounts of esters of the 1-substituted-3-fluoroalkyl-pyrazole-4-carboxylic acid of formula (I).

According to the process of the present invention, the compound of formula (I) obtained can for example be purified by distillation, especially by vacuum distillation. The compound of formula (I) can also be purified by crystallization, for example after dissolution in warm 1,1,1,3,3-pentafluorobutane (Solkane^{®}365 mfc) followed by addition of n-hexane and further cooling of the medium.

The advantage of the process is, for example, that no expensive noble metal catalyst is needed, and that no over-reduction occurs - only a selective C-Cl, C-Br or C-I cleavage is observed.

The starting compound of formula II 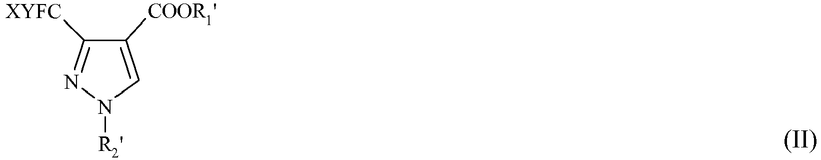 wherein
- X is Cl, Br or I
- Y is H, F or an alkyl group having from 1 to 12 carbon atoms which is optionally substituted by at least one halogen atom, an aralkyl group or an aryl group ;
- R₁' is H or an organic residue
- R₂' is H or an organic residue
may be manufactured by
(a) producing a compound of formula (IV) : XYFCC(O)CH₂C(O)OR₁' (IV) wherein R₁', X and Y are as defined above, by addition of a fluorine containing carboxylic acid chloride to ketene followed by esterification,
(b) adding an orthoformate of formula (III) : HC(OR₃)₃ (III) wherein R₃ is C₁-C₈-alkyl, C₃-C₈-cycloalkyl, C₂-C₈-alkenyl, benzyl or phenyl, to the compound of formula (IV) : XYFCC(O)CH₂C(O)OR₁ (IV) to produce an addition product of formula (V) : 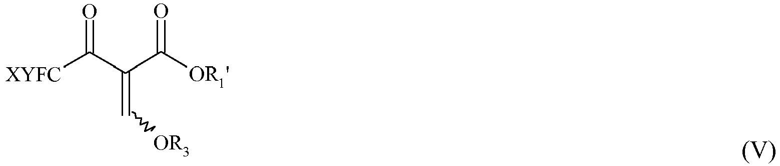 wherein R₁', R₃, X and Y are as defined above
   and
(c) reacting said addition product with a hydrazine of formula (VI) : R₂'NHNH₂ (VI) wherein R₂' is as defined above.

The definitions and preferences described above for the compounds used in the process according to the invention equally apply to the method according to the invention.

In a preferred embodiment of the method according to the invention, R₃ in the orthoformate of formula (III) is selected from the group consisting of C₁-C₄-alkyl and benzyl and in particular from the group consisting of methyl, ethyl, isopropyl and benzyl. R₃ is especially ethyl.

It has been found, surprisingly, that the method according to the invention makes it possible to prepare the compound of formula II with high regioselectivity and with a high yield.

In step (b) of the method of the manufacture of the compound of formula II of the present invention, the addition reaction of the orthoformate of formula (III) to the compound of formula (IV) can be carried out, for example, analogously to the reaction described in WO 2008/053043.

Said addition reaction also forms an alcohol R₃OH. The alcohol R₃OH is generally removed from the reaction equilibrium, for example in that it is distilled off or bound chemically. In the latter, for example the reaction can be carried out in the presence of an anhydride of a carboxylic acid, for example a C₁-C₄-alkanecarboxylic acid, such as acetic anhydride.

In the method according to the invention, the molar ratio of the orthoformate of formula (III) to the compound of formula (IV) preferably is from 1.1 to 5, and particularly preferably from 1.2:2 to about 2. Most preferably, the molar ratio is about 2.

In the method according to the invention, step (b) is generally carried out at a temperature from 80°C to 180°C, preferably from 100°C to 150°C, more preferably from 120°C to 140°C.

If desired, the compound of formula (V) is purified prior to being used in step (c) of the method according to the invention. Examples of purification steps which can be used to purify the compound of formula (V) include removal of solvents, extraction, distillation, chromatography, or a combination of these methods. It is preferred to subject the reaction mixture obtained in step (b) of the method according to the invention to a distillation.

There are alternative embodiments to produce the compound of formula (II).

For example, instead of the compound of formula (V) in step b), a compound of formula (VI), XYFC-C(O)-C(OR₃)(OR₃)-C(O)-C(O)R₁' can be applied. X, Y, R₃ and R₁' have the meaning given above. The compounds of formula (V) can be prepared, as described in unpublished European patent application N° 12162417.5, filed March 30, 2012, adding an orthoformate of formula (III) : HC(OR₃)₃ (III) wherein R₃ is C₁-C₈-alkyl, C₃-C₈-cycloalkyl, C₂-C₈-alkenyl, benzyl or phenyl, to the compound of formula (IV) : XYFCC(O)CH₂C(O)OR, (IV) in the absence of acetic acid anhydride.

There also alternative embodiments to perform step c).

For example, instead of the hydrazine of formula (VI) : R₂'NFTNH₂ (VI), a hydrazone of formula (VII), R₂'NHN=C(R₄)(R₅) wherein R4 and R5 are the same or different and are preferably C₁-C₈-alkyl, C₃-C₈-cycloalkyl, C₂-C₈-alkenyl, benzyl or phenyl. Especially preferably, R₄ and R₅ are the same or different and are C1 to C3 alkyl ; especially, both are methyl. This alternative is described in US 2011/0009642. The compounds of formula (VII) can be prepared from the substituted hydrazine of formula (VI) and a ketone R₄-C(O)-R₅.

In still another alternative of step c), instead of the monosubstituted hydrazine R₂'NHNH₂ (VI), its adduct with CO₂ (called the "adduct") can be applied. Such adducts can be prepared from the hydrazine and CO₂ as described in unpublished European patent application N° 12162419.1, filed March 30, 2012.

The method according to the invention advantageously avoids the use of expensive starting materials, in particular 2,2-difluoroacetoacetic esters. Instead, it is possible to use the much less expensive halodifluoromethyl compounds, such as 2-chloro-2,2-difluoroacetoacetic esters. For instance, 2,2-difluoroacetoacetic esters are in general prepared by the Claisen reaction. For agrochemical applications, said Claisen reaction often needs expensive bases and leads to extensive waste formation. The preparation of the compounds of general formula (IV) in step (a) by the ketene technology allows to avoid or minimize waste formation and does not need an quite expensive base. According to WO-A-2009/021987, the compounds of formula (IV) can be obtained, by addition of fluorine containing carboxylic acid chlorides to ketene followed by esterification. The used raw materials, such as difluorochloroacetyl chloride are available in industrial scale and can be produced by environmentally friendly technologies such as e.g. photochemical oxidation of 1,1-difluoro-1,2,2-trichloroethane with oxygen.

Some of the compounds of formula (IV) : XYFCC(O)CH₂C(O)OR₁ (IV), produced in step (a) of the method of the present invention are commercially available or can be prepared according to other known synthetic methods. For instance, the compounds of formula (IV) can be prepared by Claisen condensation of the corresponding fluorine containing carboxylate and acetate.

In the method according to the invention, the hydrazine of formula (VI), the hydrazone of formula (VII) or the adduct of monosubstituted hydrazine and CO₂ are preferably used in step (c) in anhydrous form.

If desired, the hydrazine or the hydrazone can be dissolved in an organic solvent, for example an organic solvent which comprises at least one halogen in such as described above in the context process of the invention. The adduct may beapplied as emulsion or suspension in an organic solvent.

In one aspect of the method of the present invention, the hydrazine, hydrazone or the adduct is added in anhydrous form to a reaction solution comprising compound (V) and an organic solvent which comprises at least one halogen.

In another aspect of the method of the present invention, the hydrazine or hydrazone dissolved in an organic solvent, or the adduct in the form of an emulsion or suspension in particular the organic solvent which comprises at least one halogen, is added to the reaction solution comprising compound (V) and the organic solvent which comprises at least one halogen.

In yet another aspect of the process of the present invention, the compound of formula (V) is added to the hydrazine, hydrazone or adduct, preferably dissolved, emulsified or suspended, respectively, in the organic solvent which comprises at least one halogen.

In an alternative and more preferred aspect of the process of the present invention, the compound (V) present in the organic solvent which comprises at least one halogen is added to the hydrazine, hydrazone or adduct, preferably dissolved or emulsified or suspended, respectively, in the organic solvent which comprises at least one halogen.

In the method according to the invention, the molar ratio of the hydrazine, hydrazone or adduct to the compound of formula (V) preferably is from 0.8 to 1.2, and particularly preferably from 0.8 to 1.0 to about 1. Most preferably, the molar ratio is about 1.

In the method according to the invention, the reaction in step (c) is generally carried out in a solvent. The solvent to be used may, for example, be a protic polar solvent, a hydrocarbon, an aliphatic hydrocarbon, an aprotic polar solvent or an ionic liquid.

Examples of suitable protic polar solvents include aliphatic alcohols having preferably 1 to 4 carbon atoms, such as methanol, ethanol, n-propanol, isopropanol, n-butanol, isobutanol or tert-butanol.

Examples of suitable hydrocarbons include aromatic hydrocarbons, aliphatic hydrocarbon or halogenated hydrocarbons.

Suitable aromatic hydrocarbons are selected, for example from benzene, toluene, xylenes, cumene, chlorobenzene, nitrobenzene and tertbutylbenzene.

Suitable aliphatic hydrocarbons are selected for example from pentane, hexane or octane.

Suitable halogenated hydrocarbons are selected for example from hydrochlorocarbons such as dichloromethane, chloroform, carbon tetrachloride or 1,2-dichloroethane, or hydro fluorocarbons such as 1,1,1,3,3-pentafluorobutane (Solkane^{®}365 mfc) or hydrochlorofluorocarbons, such as, 3,3-dichloro-1,1,1,2,2-pentafluoropropane and/or 1,3-dichloro-1,1,2,2,3-pentafluoropropane.

Examples of suitable aprotic polar solvents include ethers, amides, nitriles such as acetonitrile or propionitrile or esters such as ethyl acetate, butyl acetate or dimethyl carbonate.

Ethers may be cyclic or acyclic ethers, such as for example diethyl ether, tert-butyl methyl ether (MTBE), tetrahydrofuran (THF) or dioxane. Amides may be cyclic or acyclic, such as dimethylformamide, dimethylacetamide, N-methylpyrrolidone or tetramethylurea.

These solvents may be used alone or in combination as a mixture.

The reaction in step (c) is preferably carried out in a halogenated hydrocarbon, in particular in a hydrofluorocarbon and particularly preferably in 1,1,1,3,3-pentafluorobutane (Solkane^{®}365 mfc). The use of a hydrofluorocarbon, in particular 1,1,1,3,3-pentafluorobutane as solvent allows for particularly efficient formation of the esters of 1-substituted-3-fluoroalkyl-pyrazole-4-carboxylic acid in very high regioselectivity.

In a preferred aspect of the method of the present invention, the solvent is substantially free of water.

For the purpose of the present invention, the term "solvent substantially free of water" denotes in particular that the content of water is equal to or lower than 1 wt % by weight relative to the total weight of solvent, preferably equal to or lower than 7000 ppm, more preferably equal to or lower than 5000 ppm, most preferably equal to or lower than 2000 ppm. The solvent substantially free of water generally contains at least 1ppm by weight of water, often at least 10 ppm by weight of water relative to the total weight of solvent. Solvents which are substantially free of water allow to maintain a high reaction rate and the formation of phase separation and consequently, in general, no additional phase transfer catalysts are required.

If appropriate, the solvent is used usually in an amount of from 50 to 99 by weight, preferably from 60 to 99 % by weight, more preferably from 75 to 99 % by weight of the solvent relative to the total weight of the reaction medium.

If desired, the reaction in step (c) optionally may be carried out in the presence of a base. If a base is used, it may be an inorganic base or an organic base. When an inorganic base is used, it may be suitably selected from the group consisting of alkali metal hydroxides such as sodium hydroxide, potassium hydroxide, lithium hydroxide, cesium hydroxide, alkaline earth metal hydroxides such as calcium hydroxide, barium hydroxide, magnesium hydroxide, strontium hydroxide, and basic alkali metal salts such as sodium carbonate, sodium hydrogencarbonate, potassium carbonate and potassium hydrogencarbonate. Preferred bases are sodium hydroxide and potassium hydroxide. Most preferred base is potassium hydroxide. When an organic base is used, it may be suitably selected from the group consisting of nitrogen-containing heterocyclic compounds such as pyridine, quinoline or picoline ; and tertiary bases such as triethylamine, dimethylaniline, diethylaniline and 4-dimethylaminopyridine. Among them, pyridine, triethylamine, dimethylaniline, diethylaniline and 4-dimethylaminopyridine are preferred. A single base can be used or a mixture of several bases.

In the method according to the invention, step (c) is generally carried out at a temperature from -20°C to 60°C, preferably from 0°C to 50°C, more preferably from 10°C to 40°C. In a specific embodiment, an initial reaction temperature is set and the reaction temperature is changed during the reaction. Typical initial reaction temperatures range from -60 to 0°C, in particular from -60 to -20°C. Good results were obtained with the temperature set from -30 to -20°C. If appropriate, during the reaction the reaction mixture is warmed to a temperature of from 0 to 60°C, in particular from 10 to 40°C.

It has been found that the compounds of formula (II) are stable to decomposition but reactive towards hydrogenation.

In a most preferred aspect of the invention described herein, the compound of formula (I) is an ester of 1-methyl-3-difluoromethyl-pyrazole-4-carboxylic acid, in particular the ethyl ester.

This compound can be obtained according to the process of the present invention for example from the reduction of an ester of 1-methyl-3-chlorodifluoromethyl-pyrazole-4-carboxylic acid as compound of formula (II), in particular the ethyl ester, with a hydrogen donating compound, especially isopropanol, in the presence of UV, as described above.

In a preferred embodiment of this especially preferred process of the present invention,
(a) the 1-methyl-3-chlorodifluoromethyl-pyrazole-4-carboxylic acid as compound of formula (II), in particular the ethyl ester, is obtained from the reaction of an ester of 2-(ethoxymethylene)-4-chloro-4,4-difluoro-3-oxobutanoic acid as compound of formula (V), in particular the ethyl ester, with methylhydrazine, the hydrazone formed from methylhydrazine and acetone, or the adduct of methylhydrazine and CO₂,
(b) said 2-(ethoxymethylene)-4-chloro-4,4-difluoro-3-oxobutanoic acid is formed by the addition reaction of an orthoformate of formula (III), in particular triethyl orthoformate, to an ester of 4-chloro-4,4-difluoro-3-oxobutanoic acid as compound of formula (IV), in particular the ethyl ester.

The invention also concerns a process for the manufacture of an agrochemically or pharmaceutically active compound which comprises the use, the process or the method according to the invention. Particularly, the compound of formula (II) according to the invention can be (a) used as starting material in the process according to the invention to produce compound of formula (I) and (b) the compound of formula (I) is further reacted to manufacture an agrochemically or pharmaceutically active compound. An example of further reaction according to step (b) is illustrated in WO 2005/123 690.

Should the disclosure of any patents, patent applications, and publications referred to herein by reference conflict with the description of the present application to the extent that it may render a term unclear, the present description shall take precedence.

The following example is intended to further explain the invention without limiting it.

### Example 1 :

### Preparation of ethyl- 4,4-difluoro-4-chloro 3-oxo-butanoic acid

In a three-neck round bottom flask, chlorodifluoroacetyl chloride (148.92 g, 1 mol) was dissolved in methylene chloride (500 mL) and the solution was cooled to -30°C. During 2 hours, ketene from a ketene generator (at a rate of *ca.* 930 mmol/h) was passed through the solution of chlorodifluoroacetyl chloride. The reaction mixture was warmed up to 0°C and kept for 1 hour at 0°C. Ethanol (61.98 g, 1.94 mol) was added dropwise to the solution while keeping the temperature below 5°C. The solution was stirred for another 0.5 hour. The reaction mixture was transferred to a 2-liter flask and concentrated on a rotary evaporator under reduced pressure (30°C, 300 mBar). The residue (282.78 g) was further distilled over a 60-cm Vigreux column under a pressure of 30 mBar. Ethyl- 4,4-difluoro-4-chloro 3-oxo-butanoic acid was recovered at a temperature of 58-65°C as a colorless liquid. The yield was 85 % of the theoretical yield, and a purity of 98.0 % was obtained.

### Example 2 : Preparation of ethyl 1-methyl-3-chlorodifluoromethyl-pyrazole-4-carboxylate (CDFMMP)

A solution of ethyl 4-chloro-4,4-difluoro-3-oxo-butanoate (19 g, 95 mmol), triethyl orthoformate (28 g, 190 mmol) and acetic anhydride (29 g, 284 mmol) were heated to 120 to 140°C with continuous removal of the low boilers, like ethyl acetate produced. After 7 h the low volatility components are removed in vacuum yield more or less quantitative, although during distillation of the product variable yields are observed.

Crude ethyl 2-(ethoxymethylene)-4-chloro-4,4-difluoro-3-oxobutanoate (95 mmol) dissolved in the solvent Solkane^{®}365 mfc (200 mL)is reacted with methylhydrazine (4.9 mL, 95 mmol) under ice cooling. GC shows a ratio of 85 % CDFMMP to 15 % of the regio isomer. After 1 h at room temperature the reaction mixture is washed with 2 N HCl (100 mL) and water (100 mL). After drying with sodium sulfate, filtration and concentration under reduced pressure the desired product (14 g, 64 % over 2 steps) is isolated by column chromatography.

### Example 3 : Reduction of CDFMMP to ethyl 1-methyl-3-difluoromethyl-pyrazole-4-carboxylate (DFMMP) by reduction in the presence of an alcohol

3 g CDFMMP (0.0126 mmol) were dissolved in 120 ml of isopropanol. The resulting solution was irradiated at room temperature in a photo reactor with a 20 W low pressure UV lamp. After 2 hours, the solvent was evaporated, and the crude reaction product was analyzed by GC-MS (gas chromatography, coupled with mass spectrometry) revealing 29 % of the desired product and 62 % of unreacted starting material.

## Claims

1. A process for the manufacture of an ester of a 1-substituted-3-fluoroalkyl-pyrazole-4-carboxylic acid of formula (I) 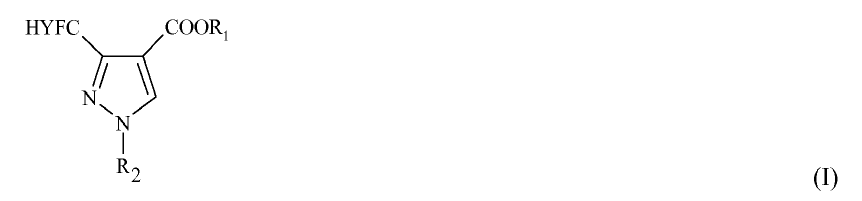 wherein
- Y is H, F or an alkyl group having from 1 to 12 carbon atoms which is optionally substituted by at least one halogen atom, an aralkyl group or an aryl group,
- R₁ is H or an organic residue,
- R₂ is H or an organic residue,
which comprises submitting a compound of formula (II) 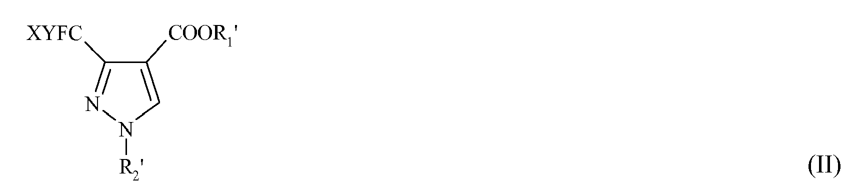 wherein Y is as defined above
- X is Cl, Br or I,
- R₁' is H or an organic residue,
- R₂' is H or an organic residue,
to a reduction reaction with a hydrogen donating compound in the presence of UV light.

2. The process according to claim 1, wherein R₁ is methyl, ethyl, n-propyl or isopropyl, preferably ethyl.

3. The process according to anyone of claims 1 to 2, wherein R₂ is methyl.

4. The process according to anyone of claims 1 to 3, wherein X is Cl and Y is F.

5. The process according to anyone of claims 1 to 4 wherein the reduction is carried out in the presence of an organic hydrogen donating compound.

6. The process according to claim 5, wherein the hydrogen donating compound is selected from the group consisting of alcohols, ethers, ketones, hydrocarbons, and mixtures of two or more thereof.

7. The process according to claims 1 to 6, wherein the hydrogen donating compound is an alcohol.

8. The process according to any one of claims 1 to 7, wherein the reduction reaction is conducted in the absence of additive, especially in the absence of additive selected from organic bases or inorganic bases.

9. Use of the compound of formula (I), obtained according to claims 1 to 8, as an intermediate in the manufacture of an agrochemically or pharmaceutically active compound.

10. A process for the manufacture of an agrochemically or pharmaceutically active compound which comprises the use according to claim 9.

## Patentansprüche

1. Verfahren zur Herstellung eines Esters einer 1-substituierten 3-Fluoralkylpyrazol-4-carbonsäure der Formel (I) 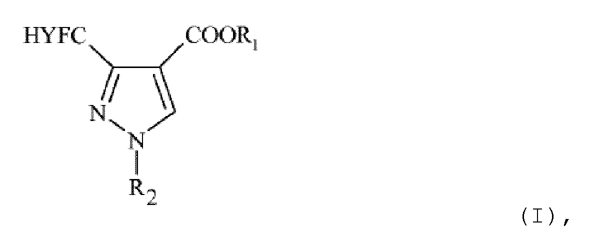 wobei
- Y für H, F oder eine Alkylgruppe mit 1 bis 12 Kohlenstoffatomen, die gegebenenfalls durch mindestens ein Halogenatom substituiert ist, eine Aralkylgruppe oder eine Arylgruppe steht,
- R₁ für H oder einen organischen Rest steht,
- R₂ für H oder einen organischen Rest steht,
bei dem man eine Verbindung der Formel (II) 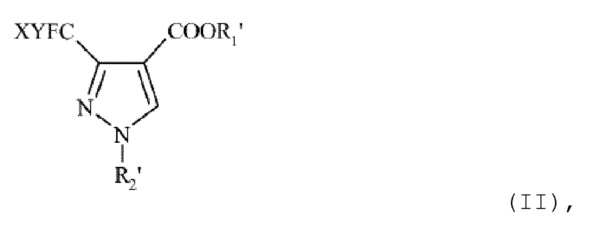 in welcher Y wie oben definiert ist,
- X für Cl, Br oder I steht,
- R₁' für H oder einen organischen Rest steht,
- R₂' für H oder einen organischen Rest steht,
einer Reduktionsreaktion mit einer wasserstoffspendenden Verbindung in Gegenwart von UV-Licht unterzieht.

2. Verfahren nach Anspruch 1, wobei R₁ für Methyl, Ethyl, n-Propyl oder Isopropyl, vorzugsweise Ethyl, steht.

3. Verfahren nach Anspruch 1 oder 2, wobei R₂ für Methyl steht.

4. Verfahren nach einem der Ansprüche 1 bis 3, wobei X für Cl und Y für F steht.

5. Verfahren nach einem der Ansprüche 1 bis 4, wobei die Reduktion in Gegenwart einer organischen wasserstoffspendenden Verbindung durchgeführt wird.

6. Verfahren nach Anspruch 5, wobei die wasserstoffspendende Verbindung aus der aus Alkoholen, Ethern, Ketonen, Kohlenwasserstoffatomen und Mischungen von zwei oder mehr davon bestehenden Gruppe ausgewählt ist.

7. Verfahren nach einem der Ansprüche 1 bis 6, wobei es sich bei der wasserstoffspendenden Verbindung um einen Alkohol handelt.

8. Verfahren nach einem der Ansprüche 1 bis 7, wobei die Reduktionsreaktion in Abwesenheit eines Zusatzstoffes, insbesondere in Abwesenheit eines Zusatzstoffes ausgewählt aus organischen Basen und anorganischen Basen, durchgeführt wird.

9. Verwendung einer nach einem der Ansprüche 1 bis 8 erhaltenen Verbindung der Formel (I) als Zwischenprodukt bei der Herstellung eines agrochemischen oder pharmazeutischen Wirkstoffs.

10. Verfahren zur Herstellung eines agrochemischen oder pharmazeutischen Wirkstoffs, welches die Verwendung nach Anspruch 9 umfasst.

## Revendications

1. Procédé de fabrication d'un ester d'un acide 3-fluoroalkyl-pyrazole-4-carboxylique substitué en position 1 de formule (I) 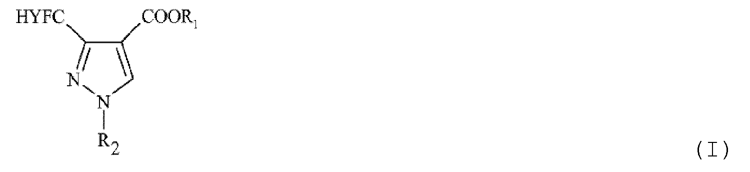 dans laquelle
- Y représente H, F ou un groupe alkyle ayant de 1 à 12 atomes de carbone qui est optionnellement substitué par au moins un atome d'halogène, un groupe aralkyle ou un groupe aryle,
- R₁ représente H ou un résidu organique,
- R₂ représente H ou un résidu organique,
qui comprend la soumission d'un composé de formule (II) 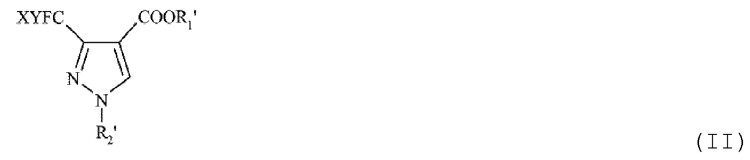 dans laquelle
- Y est tel que défini ci-dessus,
- X représente Cl, Br ou I,
- R₁' représente H ou un résidu organique,
- R₂' représente H ou un résidu organique,
à une réaction de réduction avec un composé donneur d'hydrogène en présence de rayonnement UV.

2. Procédé selon la revendication 1, dans lequel R₁ représente un groupe méthyle, éthyle, n-propyle ou isopropyle, de préférence éthyle.

3. Procédé selon l'une quelconque des revendications 1 et 2, dans lequel R₂ représente un méthyle.

4. Procédé selon l'une quelconque des revendications 1 à 3, dans lequel X représente Cl et Y représente F.

5. Procédé selon l'une quelconque des revendications 1 à 4 dans lequel la réduction est réalisée en présence d'un composé donneur d'hydrogène organique.

6. Procédé selon la revendication 5, dans lequel le composé donneur d'hydrogène est choisi dans le groupe constitué par les alcools, les éthers, les cétones, les hydrocarbures, et les mélanges d'au moins deux d'entre eux.

7. Procédé selon les revendications 1 à 6, dans lequel le composé donneur d'hydrogène est un alcool.

8. Procédé selon l'une quelconque des revendications 1 à 7, dans lequel la réaction de réduction est conduite en l'absence d'additif, en particulier en l'absence d'additif choisi parmi les bases organiques ou les bases inorganiques.

9. Utilisation du composé de formule (I), obtenu selon les revendications 1 à 8, comme intermédiaire dans la fabrication d'un composé actif sur le plan agrochimique ou pharmaceutique.

10. Procédé de fabrication d'un composé actif sur le plan agrochimique ou pharmaceutique qui comprend l'utilisation selon la revendication 9.
